# FASCICULE DE BREVET EUROPEEN

(11) **EP 0 745 426 B1**
(45) Date de publication et mention de la délivrance du brevet: **09.08.2000**
(21) Numéro de dépôt: 96201330.6
(22) Date de dépôt: 14.05.1996
(51) Int. Cl.: B01J 37/03, B01J 23/48

(54) **Procédé de préparation d'un catalyseur et son utilisation pour la conversion d'alcanes chlorés en alcènes moins chlorés**
Verfahren zur Herstellung eines Katalysators und seine Verwendung für die Umwandlung von chlorierten Alkanen in weniger chlorierte Alkenen
Catalyst preparation process and use thereof for the chlorinated alcanes conversion in less chlorinated alkenes

(30) Priorité: 24.05.1995 BE 9500468
(43) Date de publication de la demande: 04.12.1996
(73) Titulaire: SOLVAY (Société Anonyme), 1050 Bruxelles (BE)
(72) Inventeur: Delhez, Patrice, 4651 Herve (BE); Heinrichs, Benoît, 4020 Liege (BE); Pirard, Jean-Paul, 4032 Chenee (BE); Schoebrechts, Jean-Paul, 1390 Grez-Doiceau (BE)
(74) Mandataire: Jacques, Philippe

(56) Documents cités:
- EP-A- 0 177 198
- EP-A- 0 583 783
- EP-A- 0 629 442
- WO-A-93/06926
- FR-A- 2 536 410
- DATABASE WPI Section Ch, Week 8931 Derwent Publications Ltd., London, GB; Class E19, AN 89-223582 XP002011540 & JP-A-01 159 054 ( TONEN KK) , 22 Juin 1989

## Description

L'invention concerne un procédé de préparation d'un catalyseur comprenant un métal du groupe VIII et un métal du groupe Ib sur un support comprenant un oxyde inorganique, ainsi qu'un procédé de conversion d'alcanes chlorés en alcènes moins chlorés au moyen d'hydrogène en présence de ce catalyseur.

Dans la demande internationale WO-94/07827 on décrit un procédé de conversion d'alcanes chlorés en alcènes moins chlorés au moyen d'hydrogène en présence d'un catalyseur bimétallique comprenant un métal du groupe VIII et un métal du groupe Ib sur un support. Dans ce procédé connu, on préfère utiliser un support en charbon actif. Des catalyseurs comprenant un métal du groupe VIII et un métal du groupe Ib sur un support d'oxyde inorganique sont connus dans l'état de la technique. Dans un procédé connu de synthèse d'un catalyseur comprenant du palladium et de l'argent sur de la silice, on imprègne d'abord le support de silice avec une solution comprenant le palladium, on le sèche, on soumet le support comprenant le palladium à une calcination et une réduction, puis on répète la procédure avec une solution comprenant l'argent (J. Phys. Chem., 1993, vol. 97, pages 3565 à 3570, PHILLIPS J. et al.). Ce procédé connu est toutefois difficile à réaliser. Par ailleurs, l'utilisation des catalyseurs ainsi préparés ne donne pas de bons résultats dans des procédés de conversion d'alcanes chlorés en alcènes moins chlorés.

On a maintenant trouvé un procédé de préparation d'un catalyseur comprenant un métal du groupe VIII et un métal du groupe Ib sur un support comprenant un oxyde inorganique, qui est plus simple, qui ne présente pas les inconvénients décrits ci-dessus et qui confère au catalyseur des propriétés améliorées permettant notamment de convertir des alcanes chlorés en alcènes moins chlorés avec un bon rendement et une bonne sélectivité.

L'invention concerne dès lors un procédé de préparation d'un catalyseur comprenant un métal du groupe VIII et un métal du groupe Ib sur un support comprenant un oxyde inorganique, qui se caractérise en ce que :
. dans une première étape, on dissout dans un solvant organique un mélange comprenant un alcoxyde précurseur de l'oxyde inorganique, un complexe d'un composé du métal du groupe VIII et d'un composé complexant bifonctionnel répondant à la formule générale A-(CHR²)ₐ-Si(OR¹)₃ dans laquelle
   a est un nombre entier de 0 à 8,
   R¹ est une chaîne alkyle comprenant de 1 à 8 atomes de carbone,
   R² est un atome d'hydrogène ou une chaîne alkyle comprenant de 1 à 4 atomes de carbone,
   A représente un groupement donneur d'électrons capable de complexer le métal du groupe VIII,
   et un complexe d'un composé du métal du groupe Ib et d'un composé complexant bifonctionnel répondant à la formule générale B-(CHR^{2'})_{a'}-Si(OR^{1'})₃ dans laquelle
   a' est un nombre entier de 0 à 8,
   R^{1'} est une chaîne alkyle comprenant de 1 à 8 atomes de carbone,
   R^{2'} est un atome d'hydrogène ou une chaîne alkyle comprenant de 1 à 4 atomes de carbone,
   B représente un groupement donneur d'électrons capable de complexer le métal du groupe Ib;
. dans une deuxième étape, on hydrolyse le mélange afin de former un gel; et
. dans une troisième étape, on sèche le gel.

Par composé complexant bifonctionnel on entend désigner un composé comprenant au moins deux groupements fonctionnels, le premier groupement étant un groupement hydrolysable de formule -Si(OR¹)₃, respectivement -Si(OR^{1'})₃ dans laquelle R¹, respectivement R^{1'}, représentent une chaîne alkyle comprenant de 1 à 8 atomes de carbone, le deuxième groupement étant le groupement donneur d'électrons A, respectivement B. Le groupement A est un groupement donneur d'électrons capable de complexer le métal du groupe VIII. Le groupement B est un groupement donneur d'électrons capable de complexer le métal du groupe Ib. Le groupement donneur d'électrons peut notamment être choisi en fonction du métal du groupe VIII, respectivement du métal du groupe Ib, mis en oeuvre.

Avantageusement, le groupement donneur d'électrons A est sélectionné parmi ceux de formule générale R⁶R⁵N-(CHR⁴)_{b}-N(R⁷)- dans laquelle R⁴, R⁵, R⁶ et R⁷ sont, indépendamment, sélectionnés parmi un atome d'hydrogène ou une chaîne alkyle comprenant de 1 à 4 atomes de carbone, et b est un nombre entier de 1 à 4.

Avantageusement, le groupement donneur d'électrons B est sélectionné parmi ceux de formule générale R^{6'}R^{5'}N-(CHR^{4'})_{b'}-N(R^{7'})- dans laquelle R^{4'}, R^{5'}, R^{6'} et R^{7'} sont, indépendamment, sélectionnés parmi un atome d'hydrogène ou une chaîne alkyle comprenant de 1 à 4 atomes de carbone, et b' est un nombre entier de 1 à 4, et ceux de formule générale N(R^{3'})₂-dans laquelle R^{3'} est un atome d'hydrogène et/ou une chaîne alkyle comprenant de 1 à 4 atomes de carbone.

Dans le procédé selon l'invention, le métal du groupe VIII est de préférence sélectionné parmi le palladium, le platine, l'iridium, le nickel, le cobalt et le rhodium. Particulièrement préféré est le palladium.

Dans une forme d'exécution avantageuse du procédé selon l'invention, le métal du groupe VIII est le palladium et le composé complexant bifonctionnel qui lui correspond est sélectionné parmi ceux de formule générale R⁶R⁵N-(CHR⁴)_{b}-NH-(CHR²)ₐ-Si(OR¹)₃ dans laquelle
a est un nombre entier de 1 à 4,
b est un nombre entier de 1 à 3,
R¹ est une chaîne alkyle comprenant de 1 à 4 atomes de carbone,
R², R⁴, R⁵ et R⁶ sont, indépendamment, sélectionnés parmi un atome d'hydrogène et une chaîne méthyle ou éthyle. Tout particulièrement préférés sont les composés répondant à la formule générale NH₂-(CH₂)₂-NH-(CH₂)₃-Si(OR¹)₃ dans laquelle R¹ est une chaîne alkyle comprenant de 1 à 4 atomes de carbone. Dans cette forme d'exécution du précédé, le composé du palladium est de préférence un composé organique du palladium. Particulièrement préféré est l'acétylacétonate de palladium.

Dans le procédé selon l'invention, le métal du groupe Ib est de préférence sélectionné parmi le cuivre et l'argent. Particulièrement préféré est l'argent.

Dans une forme d'exécution avantageuse du procédé selon l'invention, le métal du groupe Ib est l'argent et le composé complexant bifonctionnel qui lui correspond est sélectionné parmi ceux de formule générale R^{6'}R^{5'}N-(CHR^{4'})_{b'}-NH-(CHR^{2'})_{a'}-Si(OR^{1'})₃ dans laquelle
a' est un nombre entier de 1 à 4,
b' est un nombre entier de 1 à 3,
R^{1'} est une chaîne alkyle comprenant de 1 à 4 atomes de carbone,
R^{2'}, R^{4'}, R^{5'} et R^{6'} sont, indépendamment, sélectionnés parmi un atome d'hydrogène et une chaîne méthyle ou éthyle, et ceux de formule générale N(R^{3'})₂-(CHR^{2'})_{a'}-Si(OR^{1'})₃ dans laquelle
a' est un nombre entier de 1 à 4,
R^{1'} est une chaîne alkyle comprenant de 1 à 4 atomes de carbone,
R^{2'} et R^{3'} sont, indépendamment, sélectionnés parmi un atome d'hydrogène et une chaîne méthyle ou éthyle. Tout particulièrement préférés sont les composés répondant à la formule générale NH₂-(CH₂)₂-NH-(CH₂)₃-Si(OR^{1'})₃
ou à la formule générale NH₂-(CH₂)₃-Si(OR^{1'})₃ dans lesquelles R^{1'} est une chaîne alkyle comprenant de 1 à 4 atomes de carbone. Dans cette forme d'exécution du procédé, le composé de l'argent est de préférence un composé organique de l'argent. Particulièrement préféré est l'acétate d'argent.

Dans le procédé selon l'invention, l'alcoxyde précurseur de l'oxyde inorganique est de préférence sélectionné parmi les alcoxydes de silicium, d'aluminium, de titane et de zirconium. Selon une variante du procédé selon l'invention, on peut mettre en oeuvre un mélange d'alcoxydes. De bons résultats ont été obtenus avec des alcoxydes de silicium répondant à la formule générale Si(OR⁸)₄ dans laquelle R⁸ est une chaîne alkyle comprenant de 1 à 6 atomes de carbone.

Dans le procédé selon l'invention, le solvant organique a comme fonction de dissoudre l'alcoxyde précurseur de l'oxyde inorganique, le complexe d'un composé du métal du groupe VIII et le complexe d'un composé du métal du groupe Ib afin de former une solution homogène. Le solvant organique est de préférence sélectionné parmi les alcools. Particulièrement préférés sont les alcools inférieurs comprenant moins de 5 atomes de carbone.

Dans la première étape du procédé selon l'invention, on dissout dans le solvant organique, un mélange comprenant l'alcoxyde précurseur de l'oxyde inorganique, le complexe d'un composé du métal du groupe VIII et le complexe d'un composé du métal du groupe Ib. L'ordre selon lequel ces trois composés sont introduits dans le solvant organique n'est pas critique, ni les quantités respectives mises en oeuvre, à condition d'obtenir une solution homogène. Les quantités relatives des complexes et de l'alcoxyde précurseur mises en oeuvre vont déterminer les quantités relatives du métal du groupe VIII et du métal du groupe Ib sur le support comprenant l'oxyde inorganique. Les quantités mises en oeuvre peuvent varier dans de larges limites. Habituellement, on règle les quantités des complexes et de l'alcoxyde précurseur pour obtenir un catalyseur comprenant au moins 0,05 % en poids du métal du groupe VIII. De préférence, on règle ces quantités de façon à obtenir un catalyseur comprenant au moins 0,5 % en poids du métal du groupe VIII. Elles sont habituellement réglées de façon à obtenir un catalyseur dont la quantité en métal du groupe VIII ne dépasse pas 15 % en poids. De préférence, on règle ces quantités de façon à obtenir un catalyseur dont la quantité en métal du groupe VIII ne dépasse pas 10 % en poids. D'une façon particulièrement préférée, les quantités mises en oeuvre sont choisies pour obtenir un catalyseur comprenant 5 % ou moins de métal du groupe VIII. Habituellement, on règle ces quantités pour obtenir un catalyseur comprenant au moins 0,05 % en poids du métal du groupe Ib. De préférence, on règle ces quantités de façon à obtenir un catalyseur comprenant au moins 0,5 % en poids du métal du groupe Ib. Ces quantités sont habituellement réglées de façon à obtenir un catalyseur dont la quantité en métal du groupe Ib ne dépasse pas 15 % en poids. De préférence, on règle ces quantités de façon à obtenir un catalyseur dont la quantité en métal du groupe Ib ne dépasse pas 10 % en poids. D'une façon particulièrement préférée, les quantités mises en oeuvre sont choisies pour obtenir un catalyseur comprenant 5 % ou moins de métal du groupe Ib.

Les quantités relatives du complexe d'un composé du métal du groupe VIII et du complexe d'un composé du métal du groupe Ib vont déterminer le rapport pondéral du métal du groupe VIII au métal du groupe Ib dans le catalyseur. Les quantités relatives de ces deux complexes sont de préférence réglées de façon à obtenir un catalyseur dont le rapport pondéral du métal du groupe VIII au métal du groupe Ib est d'au moins 0,1. Ces quantités sont d'une manière particulièrement préférée, réglées afin d'obtenir un rapport pondéral d'au moins 0,4. Elles sont de préférence réglées de façon à obtenir un catalyseur dont le rapport pondéral du métal du groupe VIII au métal du groupe Ib ne dépasse pas 10. Ces quantités sont d'une manière particulièrement préférée, réglées afin d'obtenir un rapport pondéral qui ne dépasse pas 2,5.

Dans la deuxième étape du procédé selon l'invention, on hydrolyse le mélange obtenu à la première étape, au moyen d'eau afin d'obtenir un gel. La quantité d'eau mise en oeuvre dans cette deuxième étape doit être au moins suffisante pour hydrolyser l'alcoxyde précurseur et les groupements hydrolysables de formule -Si(OR¹)₃, respectivement -Si(OR^{1'})₃ des composés complexants bifonctionnels des complexes des composés des métaux du groupe VIII et Ib, respectivement. D'une manière préférée, on met en oeuvre un léger excès d'eau. L'eau d'hydrolyse mise en oeuvre dans la deuxième étape du procédé se trouve habituellement à un pH égal ou supérieur à 7. Ce pH peut par exemple être obtenu en mettant l'eau d'hydrolyse en oeuvre à l'état d'une solution aqueuse ammoniacale. L'hydrolyse est avantageusement effectuée en ajoutant l'eau d'hydrolyse, lentement et sous agitation, au mélange préparé à la première étape du procédé. L'eau peut éventuellement être diluée au moyen d'un solvant organique, de préférence le même solvant organique que celui mis en oeuvre dans la première étape du procédé. Après l'ajout de l'eau au mélange issu de la première étape, le milieu réactionnel est avantageusement conservé dans un récipient fermé, de préférence à une température supérieure à 50 °C, pendant une durée au moins suffisante pour former un gel, habituellement une durée variant de quelques heures à quelques jours.

Dans la troisième étape du procédé selon l'invention, on sèche le gel obtenu à la deuxième étape du procédé. Le séchage a pour fonction d'éliminer le solvant du gel.

Selon une première variante d'exécution de la troisième étape du procédé, le gel est séché dans des conditions supercritiques. Par conditions supercritiques, on entend désigner des conditions dans lesquelles la température et la pression sont au dessus de la température critique et de la pression critique du liquide présent dans le gel. Ce liquide comprend essentiellement le solvant organique mis en oeuvre, l'eau excédentaire et les produits résiduels de l'hydrolyse. Le séchage du gel dans des conditions supercritiques permet d'obtenir un catalyseur comprenant le support sous la forme d'un aérogel.

Selon une autre variante d'exécution de la troisième étape du procédé, qui est préférée, le gel est séché sous une pression inférieure à la pression atmosphérique. Dans cette variante du procédé, la pression et la température de séchage peuvent varier dans de larges limites. La température de séchage est de préférence supérieure ou égale à 50 °C. De bons résultats ont été obtenus en séchant le gel à une température comprise entre 60 °C et 200 °C, sous une pression d'environ 10 mbar, pendant plusieurs jours. Le séchage du gel dans ces conditions permet d'obtenir un catalyseur comprenant le support sous la forme d'un xérogel.

Le procédé selon l'invention pour la préparation d'un catalyseur peut avantageusement comprendre une quatrième étape qui consiste à calciner le gel séché issu de la troisième étape du procédé. La calcination a pour but d'éliminer les résidus organiques du gel. La calcination peut avantageusement être effectuée dans un flux d'oxygène ou d'air, à une température supérieure ou égale à 200 °C et ne dépassant pas 800 °C.

Le procédé selon l'invention pour la préparation d'un catalyseur peut avantageusement également comprendre une étape ultérieure de traitement sous une atmosphère réductrice, pour obtenir les métaux du groupe VIII et IB à l'état élémentaire dans le catalyseur. Cette réduction consiste, habituellement, à traiter le gel séché sous une atmosphère réductrice telle que par exemple d'hydrogène, à une température d'au moins 100 °C et de préférence inférieure ou égale à 500 °C.

Le procédé selon l'invention permet d'obtenir une dispersion homogène des métaux des groupes VIII et Ib, à l'état finement divisé, de dimensions de l'ordre de quelques nanomètres, dans le support comprenant l'oxyde inorganique. Le procédé de préparation selon l'invention permet notamment d'obtenir les métaux du groupe VIII et Ib sous la forme d'un alliage sur le support.

Dès lors, l'invention concerne également un catalyseur comprenant un métal du groupe VIII et un métal du groupe Ib sur un support comprenant un oxyde inorganique susceptible d'être obtenu au moyen du procédé de préparation selon l'invention, décrit ci-dessus.

Le catalyseur issu du procédé de préparation selon l'invention peut être utilisé dans un grand nombre de procédés industriels. Il convient spécialement bien pour des réactions d'hydrogénation. Le catalyseur issu du procédé de préparation selon l'invention est particulièrement bien adapté pour convertir des alcanes chlorés en alcènes moins chlorés, au moyen d'hydrogène.

Dès lors, l'invention concerne également un procédé de conversion d'alcanes chlorés en alcènes moins chlorés, au moyen d'hydrogène, qui se caractérise en ce qu'on met en oeuvre un catalyseur susceptible d'être obtenu au moyen du procédé de préparation selon l'invention, décrit ci-dessus.

Les alcanes chlorés mis en oeuvre dans le procédé selon l'invention sont des alcanes comprenant au moins 1 atome de chlore. Les alcanes chlorés acycliques sont préférés. Les alcanes chlorés acycliques, de formule générale CₙH₂ₙ₊₂₋ₓClₓ dans laquelle n = 2 à 6 et x = 1 à (2n+2) sont spécialement avantageux. Particulièrement préférés sont les chloroéthanes et les chloropropanes, et plus spécialement les dichloroéthanes, les dichloropropanes et les trichloropropanes. Tout particulièrement préféré est le 1,2-dichloroéthane et le 1,2-dichloropropane.

Par alcènes moins chlorés on entend désigner les alcènes dont le nombre d'atomes de carbone correspond au nombre d'atomes de carbone de l'alcane chloré mis en oeuvre et qui ont au moins 1 atome de chlore en moins que l'alcane chloré mis en oeuvre. L'alcène moins chloré tel que défini dans la présente invention peut donc ne contenir aucun atome de chlore. Dans le cas d'alcanes chlorés de formule générale CₙH₂ₙ₊₂₋ₓClₓ dans laquelle x = 1 à (2n+2) , les alcènes formés répondent donc à la formule générale CₙH_{2n-y}Cl_{y} dans laquelle y peut varier de 0 à (x-1).

Dans le procédé selon l'invention, l'hydrogène a pour fonction de convertir l'alcane chloré en un alcène moins chloré, comme exposé plus haut. L'hydrogène peut éventuellement être mélangé à un autre gaz, inerte dans les conditions de la réaction de conversion de l'alcane chloré en alcène moins chloré. Comme autre gaz, on peut utiliser un gaz du groupe des gaz inertes proprement dit, tel que l'hélium, ou un gaz qui n'intervient pas dans la réaction susdite, tel qu'un alcène. La quantité volumique d'hydrogène est de préférence d'au moins 5 % du volume total d'hydrogène et de l'autre gaz.

Le rapport molaire hydrogène/alcane chloré est de préférence d'au moins 0,1, plus particulièrement d'au moins 0,5. Ce rapport ne dépasse de préférence pas 40. D'une manière particulièrement préférée, il ne dépasse pas 20.

Le procédé selon l'invention peut être effectué en phase liquide ou en phase gazeuse. Le procédé selon l'invention est de préférence exécuté en phase gazeuse. Le procédé s'effectue de préférence à une température d'au moins 150 °C, plus particulièrement d'au moins 200 °C. La température ne dépasse habituellement pas 450 °C. De préférence elle ne dépasse pas 400 °C. La pression à laquelle est effectuée le procédé n'est pas critique en elle-même. Habituellement, on opère sous une pression d'au moins 1 bar. Généralement, la pression ne dépasse pas 30 bar. De préférence, elle ne dépasse pas 10 bar.

Le catalyseur mis en oeuvre dans le procédé selon l'invention peut être n'importe quel catalyseur susceptible d'être obtenu au moyen du procédé de préparation selon l'invention, décrit ci-dessus. D'une manière préférée, on met en oeuvre un catalyseur comprenant du palladium et de l'argent. La quantité de palladium dans le catalyseur est avantageusement d'au moins 0,05 %, de préférence d'au moins 0,5 % en poids. Habituellement, la quantité de palladium ne dépasse pas 10 % en poids. De préférence, elle ne dépasse pas 5 %. La quantité d'argent sur le support est avantageusement d'au moins 0,05 %, de préférence d'au moins 0,5 % en poids. Habituellement, la quantité d'argent ne dépasse pas 10 % en poids. De préférence, elle ne dépasse pas 5 %. Le rapport pondéral du palladium à l'argent est de préférence d'au moins 0,1. D'une manière particulièrement préférée, ce rapport pondéral est d'au moins 0,4. De préférence, le rapport pondéral palladium/argent ne dépasse pas 10. D'une manière particulièrement préférée, ce rapport ne dépasse pas 2,5. Le rapport pondéral palladium/argent est avantageusement sélectionné de manière à obtenir un alliage palladium/argent sur le support. D'une manière préférée, on met en oeuvre un catalyseur comprenant le support sous la forme d'un xérogel.

Le procédé selon l'invention de conversion d'alcanes chlorés en alcènes moins chlorés permet d'obtenir de haut taux de transformation d'alcanes chlorés et des sélectivités élevées pour les alcènes moins chlorés recherchés, sans formation notable d'alcanes ou d'alcanes chlorés. Le procédé selon l'invention présente en plus l'avantage que la désactivation du catalyseur au cours du temps est particulièrement lente et que la régénération du catalyseur peut se faire facilement, notamment au moyen d'air.

Le procédé selon l'invention trouve une application intéressante dans la conversion de chloropropanes en propylène, et plus particulièrement de chloropropanes formés comme sous-produits dans la fabrication du chlorure d'allyle par chloration du propylène et/ou dans la fabrication d'épichlorhydrine par hypochloration du chlorure d'allyle. Des exemples de chloropropanes sous-produits de ces fabrications sont notamment le 1,2-dichloroproet le 1,2,3-trichloropropane. Cette application particulière du procédé selon l'invention est particulièrement avantageuse car elle permet d'obtenir le propylène avec une grande sélectivité, permettant son recyclage à l'étape de chloration du propylène en chlorure d'allyle.

L'invention se trouve plus amplement illustrée par les exemples suivants.

### Example 1 (préparation d'un catalyseur, conformément à l'invention)

On a préparé trois solutions distinctes :
. solution no. 1 : sous agitation magnétique, on a ajouté 1,2 ml de 3-(2-aminoéthylamino)-propyltriméthoxysilane à une solution comprenant 0,8417 g d'acétylacétonate de palladium et 45,5 ml d'éthanol, jusqu'à l'obtention d'une solution limpide.
. solution no. 2 :sous agitation magnétique, on a ajouté 2,6 ml de 3-aminopropyltriéthoxysilane à une solution comprenant 0,9143 g d'acétate d'argent et 45,5 ml d'éthanol, jusqu'à l'obtention d'une solution limpide.
. solution no. 3 : on a ajouté 91 ml d'éthanol à 27,8 ml d'une solution aqueuse 0,18 M NH₃.

Sous agitation, on a versé la solution no. 2 dans un réacteur contenant la solution no. 1, puis 66,3 ml de tetraéthylorthosilicate ont été ajoutés. Au mélange obtenu on a ajouté, goutte à goutte et sous une forte agitation, la solution no. 3 comprenant l'eau. Le réacteur a été fermé de manière étanche et il a été placé dans une étuve chauffée à 70 °C. Après environ 15 minutes, on a observé la formation d'un gel. Le gel a été conservé dans l'étuve.

Après 3 jours de vieillissement, le gel a été séché dans une étuve, maintenue sous vide (pression 12 mbar) et chauffée à 80 °C pendant 2 jours, puis à 150 °C pendant 3 jours.

Le xérogel obtenu a été calciné dans un calcinateur sous un flux d'air à 400 °C pendant 8 h, puis traité au moyen d'hydrogène à 350 °C, pendant 3 h.

Le catalyseur obtenu s'est révélé comprendre 1,5 % en poids de palladium et 3 % en poids d'argent. Les analyses par diffraction X et par microscopie électronique ont montré que les métaux étaient finement dispersés sur le support, la taille des grains étant d'environ 3 nm. L'analyse par diffraction X du catalyseur a montré que les métaux étaient présents sous la forme d'un alliage comprenant 63 % atomique d'argent et 37 % atomique de palladium.

### Exemple 2 (préparation d'un catalyseur, conformément à l'invention)

Le mode opératoire décrit à l'exemple 1 a été répété en mettant en oeuvre une solution no. 2 comprenant la moitié seulement de 3-aminopropyltriéthoxysilane et d'acétate d'argent.

Le catalyseur obtenu s'est révélé comprendre 1,5 % en poids de palladium et 1,5 % en poids d'argent. Les analyses par diffraction X et par microscopie électronique ont montré que les métaux étaient finement dispersés sur le support, la taille des grains étant d'environ 3 nm. L'analyse par diffraction X du catalyseur a montré que les métaux étaient présents sous la forme d'un alliage comprenant 47 % atomique d'argent et 53 % atomique de palladium.

### Exemple 3 (préparation d'un catalyseur conformément à l'invention)

Le mode opératoire décrit à l'exemple 1 a été répété en mettant en oeuvre une solution no. 2 comprenant un quart seulement de 3-aminopropyltriéthoxysilane et d'acétate d'argent.

Le catalyseur obtenu s'est révélé comprendre 1,5 % en poids de palladium et 0,75 % en poids d'argent. Les analyses par diffraction X et par microscopie électronique ont montré que les métaux étaient finement dispersés sur le support, la taille des grains étant d'environ 2,5 nm. L'analyse par diffraction X du catalyseur a montré que les métaux étaient présents sous la forme d'un alliage comprenant 34 % atomique d'argent et 66 % atomique de palladium.

### Exemple 4 (conforme à l'invention)

Le catalyseur obtenu à l'exemple 2 a été utilisé pour convertir du 1,2-dichloroéthane en éthylène. A cet effet, 0,11 g du catalyseur de l'exemple 2 ont été introduits dans un réacteur tubulaire. Le réacteur contenant le catalyseur a ensuite été alimenté à raison de 1 l/h (Nl/h) de 1,2-dichloroéthane, de 2 l/h (Nl/h) d'hydrogène et de 37 l/h (Nl/h) d'hélium, à 300 °C, sous 3 bar. Le temps de contact moyen, c'est-à-dire le rapport entre le volume occupé par le catalyseur et le débit total d'alimentation, a été évalué à 0,13 s.

Le taux de conversion du 1,2-dichloroéthane fut de 8 % et la sélectivité en éthylène (définie comme la fraction molaire du 1,2-dichloroéthane ayant réagi, qui est transformée en éthylène) de 100 %.

### Exemple 5 (de référence)

L'exemple 4 a été répété en mettant en oeuvre 0,25 g d'un catalyseur comprenant 1,5 % en poids de palladium et 1,5 % en poids d'argent, non-conforme à l'invention, préparé par la technique d'imprégnation connue, décrite dans J. Phys. Chem., 1993, vol. 97, pages 3565 à 3570, PHILLIPS J. et al. A cet effet, pour préparer le catalyseur, on a imprégné un support en silice (GRACE® grade 1352) au moyen de solutions de Pd(NH₃)₄(OH)₂ et de AgNO₃.

Le taux de conversion du 1,2-dichloroéthane fut de 14 % et la sélectivité pour l'éthylène de 85 %.

En comparant les résultats obtenus respectivement aux exemples 4 et 5, dans les mêmes conditions opératoires et avec des catalyseurs comprenant la même quantité de palladium et d'argent, on observe que le catalyseur obtenu au moyen du procédé selon l'invention (Exemple 4) est nettement plus sélectif envers l'éthylène que le catalyseur préparé selon la technique de l'art antérieur (Exemple 5).

### Exemple 6 (conforme à l'invention)

Le catalyseur obtenu à l'exemple 2 a été utilisé pour convertir du 1,2-dichloropropane en propylène. A cet effet, 0,28 g du catalyseur de l'exemple 2 ont été introduits dans un réacteur tubulaire. Le réacteur contenant le catalyseur a ensuite été alimenté à raison de 0,75 l/h (Nl/h) de 1,2-dichloropropane, de 1,5 l/h (Nl/h) d'hydrogène et de 5,25 l/h (Nl/h) d'hélium, à 300 °C, sous 3 bar. Le temps de séjour a été évalué à 1,7 s.

Le taux de conversion du 1,2-dichloropropane fut de 25 % et la sélectivité pour le propylène de 89 %.

## Revendications

1. Procédé de préparation d'un catalyseur comprenant un métal du groupe VIII et un métal du groupe Ib sur un support comprenant un oxyde inorganique, caractérisé en ce que :
. dans une première étape, on dissout dans un solvant organique, un mélange comprenant un alcoxyde précurseur de l'oxyde inorganique, un complexe d'un composé du métal du groupe VIII et d'un composé complexant bifonctionnel répondant à la formule générale A-(CHR²)ₐ-Si(OR¹)₃ dans laquelle
a est un nombre entier de 0 à 8,
R¹ est une chaîne alkyle comprenant de 1 à 8 atomes de carbone,
R² est un atome d'hydrogène ou une chaîne alkyle comprenant de 1 à 4 atomes de carbone,
A représente un groupement donneur d'électrons capable de complexer le métal du groupe VIII,
et un complexe d'un composé du métal du groupe Ib et d'un composé complexant bifonctionnel répondant à la formule générale B-(CHR^{2'})_{a'}-Si(OR^{1'})₃ dans laquelle
a' est un nombre entier de 0 à 8,
R^{1'} est une chaîne alkyle comprenant de 1 à 8 atomes de carbone,
R^{2'} est un atome d'hydrogène ou une chaîne alkyle comprenant de 1 à 4 atomes de carbone,
B représente un groupement donneur d'électrons capable de complexer le métal du groupe Ib;
. dans une deuxième étape, on hydrolyse le mélange afin de former un gel; et
. dans une troisième étape, on sèche le gel.

2. Procédé selon la revendication 1, caractérisé en ce que le métal du groupe VIII est le palladium et le métal du groupe Ib est l'argent.

3. Procédé selon la revendication 1 ou 2, caractérisé en ce que le groupement donneur d'électrons A est un groupement sélectionné parmi ceux de formule générale R⁶R⁵N-(CHR⁴)_{b}-N(R⁷)- dans laquelle R⁴, R⁵, R⁶ et R⁷ sont, indépendamment, sélectionnés parmi un atome d'hydrogène ou une chaîne alkyle comprenant de 1 à 4 atomes de carbone, et b est un nombre entier de 1 à 4, et le groupement donneur d'électrons B est un groupement sélectionné parmi ceux de formule générale R^{6'}R^{5'}N-(CHR^{4'})_{b'}-N(R^{7'})- dans laquelle R^{4'}, R^{5'}, R^{6'} et R^{7'} sont, indépendamment, sélectionnés parmi un atome d'hydrogène ou une chaîne alkyle comprenant de 1 à 4 atomes de carbone, et b' est un nombre entier de 1 à 4, et ceux de formule générale N(R^{3'})₂- dans laquelle R^{3'} est un atome d'hydrogène ou une chaîne alkyle comprenant de 1 à 4 atomes de carbone.

4. Procédé selon la revendication 3, caractérisé en ce que le métal du groupe VIII est du palladium et le composé complexant de ce métal répond à la formule générale R⁶R⁵N-(CHR⁴)_{b}-NH-(CHR²)ₐ-Si(OR¹)₃ dans laquelle
a est un nombre entier de 1 à 4,
b est un nombre entier de 1 à 3,
R¹ est une chaîne alkyle comprenant de 1 à 4 atomes de carbone,
R², R⁴, R⁵ et R⁶ sont, indépendamment, sélectionnés parmi un atome d'hydrogène et une chaîne méthyle ou éthyle.

5. Procédé selon la revendication 4, caractérisé en ce que le composé du métal du groupe VIII est l'acétylacétonate de palladium et le composé complexant de ce métal répond à la formule générale NH₂-(CH₂)₂-NH-(CH₂)₃-Si(OR¹)₃.

6. Procédé selon l'une quelconque des revendications 3 à 5, caractérisé en ce que le métal du groupe Ib est l'argent et le composé complexant de ce métal répond à la formule générale R^{6'}R^{5'}N-(CHR^{4'})_{b'}-NH-(CHR^{2'})_{a'}-Si(OR^{1'})₃ ou à la formule générale N(R^{3'})₂-(CHR^{2'})_{a'}-Si(OR^{1'})₃ dans lesquelles
a' est un nombre entier de 1 à 4,
b' est un nombre entier de 1 à 3,
R^{1'} est une chaîne alkyle comprenant de 1 à 4 atomes de carbone,
R^{2'}, R^{3'}, R^{4'}, R^{5'} et R^{6'} sont, indépendamment, sélectionnés parmi un atome d'hydrogène et une chaîne méthyle ou éthyle.

7. Procédé selon la revendication 6, caractérisé en ce que le composé du métal du groupe Ib est l'acétate d'argent et le composé complexant de ce métal répond à la formule générale NH₂-(CH₂)₂-NH-(CH₂)₃-Si(OR^{1'})₃ ou à la formule générale NH₂-(CH₂)₃-Si(OR^{1'})₃.

8. Procédé selon l'une quelconque des revendications 1 à 7, caractérisé en ce que l'alcoxyde précurseur de l'oxyde inorganique est sélectionné parmi les alcoxydes de silicium, d'aluminium, de titane et de zirconium.

9. Procédé selon l'une quelconque des revendications 1 à 8, caractérisé en ce qu'on règle les quantités respectives des complexes et de l'alcoxyde précurseur pour obtenir un catalyseur contenant de 0,05 % à 15 % en poids du métal du groupe VIII et de 0,05 % à 15 % en poids du métal du groupe Ib.

10. Procédé selon l'une quelconque des revendications 1 à 9, caractérisé en ce qu'on règle les quantités respectives des complexes pour obtenir un catalyseur dont le rapport pondéral du métal du groupe VIII au métal du groupe Ib est de 0,1 à 10.

11. Procédé selon l'une quelconque des revendications 1 à 10, caractérisé en ce que dans la troisième étape on sèche le gel sous une pression inférieure à la pression atmosphérique afin d'obtenir un xérogel.

12. Catalyseur comprenant un métal du groupe VIII et un métal du groupe Ib sur un support comprenant un oxyde inorganique, susceptible d'être obtenu au moyen du procédé selon l'une quelconque des revendications 1 à 11.

13. Procédé de conversion d'alcanes chlorés en alcènes moins chlorés, au moyen d'hydrogène, caractérisé en ce qu'on met en oeuvre un catalyseur selon la revendication 12.

14. Procédé selon la revendication 13, caractérisé en ce que les alcanes chlorés sont choisis parmi les chloroéthanes et les chloropropanes.

## Patentansprüche

1. Verfahren zur Herstellung eines Katalysators, der ein Metall der Gruppe VIII und ein Metall der Gruppe Ib auf einem Träger, der ein anorganisches Oxid umfasst, umfasst, dadurch gekennzeichnet, dass:
- man in einem ersten Schritt ein Gemisch, das eine Alkoxid-Vorstufe des anorganischen Oxids, einen Komplex einer Verbindung des Metalls der Gruppe VIII und einer bifunktionellen komplexierenden Verbindung, die der allgemeinen Formel A-(CHR²)ₐ-Si(OR¹)₃ entspricht, in der
a eine ganze Zahl von 0 bis 8 ist,
R¹ eine Alkylkette mit 1 bis 8 Kohlenstoffatomen ist,
R² ein Wasserstoffatom oder eine Alkylkette mit 1 bis 4 Kohlenstoffatomen ist,
A eine Elektronendonorgruppe darstellt, die das Metall der Gruppe VIII komplexieren kann,
und einen Komplex einer Verbindung des Metalls der Gruppe Ib und einer bifunktionellen komplexierenden Verbindung, die der allgemeinen Formel B-(CHR^{2'})_{a'}-Si(OR^{1'})₃ entspricht, in der
a' eine ganze Zahl von 0 bis 8 ist,
R^{1'} eine Alkylkette mit 1 bis 8 Kohlenstoffatomen ist,
R^{2'} ein Wasserstoffatom oder eine Alkylkette mit 1 bis 4 Kohlenstoffatomen ist,
B eine Elektronendonorgruppe darstellt, die das Metall der Gruppe Ib komplexieren kann,
umfasst, in einem organischen Lösungsmittel auflöst,
- man in einem zweiten Schritt das Gemisch hydrolysiert, um ein Gel zu bilden, und
- man in einem dritten Schritt das Gel trocknet.

2. Verfahren gemäß Anspruch 1, dadurch gekennzeichnet, dass das Metall der Gruppe VIII Palladium ist und das Metall der Gruppe Ib Silber ist.

3. Verfahren gemäß Anspruch 1 oder 2, dadurch gekennzeichnet, dass die Elektronendonorgruppe A eine Gruppe ist, die unter denen der allgemeinen Formel R⁶R⁵N-(CHR⁴)_{b}-N(R⁷)-, in der R⁴, R⁵, R⁶ und R⁷ unabhängig voneinander unter einem Wasserstoffatom oder einer Alkylkette mit 1 bis 4 Kohlenstoffatomen ausgewählt sind und b eine ganze Zahl von 1 bis 4 ist, ausgewählt ist, und die Elektronendonorgruppe B eine Gruppe ist, die unter denen der allgemeinen Formel R^{6'}R^{5'}N-(CHR^{4'})_{b'}-N(R^{7'})-, in der R^{4'}, R^{5'}, R^{6'} und R^{7'} unabhängig voneinander unter einem Wasserstoffatom oder einer Alkylkette mit 1 bis 4 Kohlenstoffatomen ausgewählt sind und b' eine ganze Zahl von 1 bis 4 ist, und denen der allgemeinen Formel N(R^{3'})₂₋, in der R^{3'} ein Wasserstoffatom oder eine Alkylkette mit 1 bis 4 Kohlenstoffatomen ist, ausgewählt ist.

4. Verfahren gemäß Anspruch 3, dadurch gekennzeichnet, dass das Metall der Gruppe VIII Palladium ist und die komplexierende Verbindung für dieses Metall der allgemeinen Formel R⁶R⁵N-(CHR⁴)_{b}-NH-(CHR²)ₐ-Si(OR¹)₃ entspricht, in der
a eine ganze Zahl von 1 bis 4 ist,
b eine ganze Zahl von 1 bis 3 ist,
R¹ eine Alkylkette mit 1 bis 4 Kohlenstoffatomen ist,
R², R⁴, R⁵ und R⁶ unabhängig voneinander unter einem Wasserstoffatom und einer Methyl- oder Ethylkette ausgewählt sind.

5. Verfahren gemäß Anspruch 4, dadurch gekennzeichnet, dass die Verbindung des Metalls der Gruppe VIII Palladiumacetylacetonat ist und die komplexierende Verbindung für dieses Metall der allgemeinen Formel NH₂-(CH₂)₂-NH-(CH₂)₃-Si(OR¹)₃ entspricht.

6. Verfahren gemäß einem der Ansprüche 3 bis 5, dadurch gekennzeichnet, dass das Metall der Gruppe Ib Silber ist und die komplexierende Verbindung für dieses Metall der allgemeinen Formel R^{6'}R^{5'}N-(CHR^{4'})_{b'}-NH-(CHR^{2'})_{a'}-Si(OR^{1'})₃ oder der allgemeinen Formel N(R^{3'})₂-(CHR²)_{a'}-Si(OR^{1'})₃ entspricht, in denen
a' eine ganze Zahl von 1 bis 4 ist,
b' eine ganze Zahl von 1 bis 3 ist,
R^{1'} eine Alkylkette mit 1 bis 4 Kohlenstoffatomen ist,
R^{2'}, R^{3'}, R^{4'}, R^{5'} und R^{6'} unabhängig voneinander unter einem Wasserstoffatom und einer Methyl- oder Ethylkette ausgewählt sind.

7. Verfahren gemäß Anspruch 6, dadurch gekennzeichnet, dass die Verbindung des Metalls der Gruppe Ib Silberacetat ist und die komplexierende Verbindung für dieses Metall der allgemeinen Formel NH₂-(CH₂)₂-NH-(CH₂)₃-Si(OR^{1'})₃ oder der allgemeinen Formel NH₂-(CH₂)₃-Si(OR^{1'})₃ entspricht.

8. Verfahren gemäß einem der Ansprüche 1 bis 7, dadurch gekennzeichnet, dass die Alkoxid-Vorstufe des anorganischen Oxids unter den Alkoxiden von Silicium, Aluminium, Titan und Zirkonium ausgewählt ist.

9. Verfahren gemäß einem der Ansprüche 1 bis 8, dadurch gekennzeichnet, dass man die jeweiligen Mengen der Komplexe und der Alkoxid-Vorstufe einstellt, um einen Katalysator zu erhalten, der 0,05 Gew.-% bis 15 Gew.-% des Metalls der Gruppe VIII und 0,05 Gew.-% bis 15 Gew.-% des Metalls der Gruppe Ib enthält.

10. Verfahren gemäß einem der Ansprüche 1 bis 9, dadurch gekennzeichnet, dass man die jeweiligen Mengen der Komplexe einstellt, um einen Katalysator zu erhalten, bei dem das Gewichtsverhältnis des Metalls der Gruppe VIII zu dem Metall der Gruppe Ib 0,1 bis 10 beträgt.

11. Verfahren gemäß einem der Ansprüche 1 bis 10, dadurch gekennzeichnet, dass man im dritten Schritt das Gel unter einem Druck, der niedriger als der Atmosphärendruck ist, trocknet, um ein Xerogel zu erhalten.

12. Katalysator, der ein Metall der Gruppe VIII und ein Metall der Gruppe Ib auf einem Träger, der ein anorganisches Oxid umfasst, umfasst, der mittels des Verfahrens gemäß einem der Ansprüche 1 bis 11 erhalten werden kann.

13. Verfahren zur Umwandlung von chlorierten Alkanen in weniger chlorierte Alkene mittels Wasserstoff, dadurch gekennzeichnet, dass man einen Katalysator gemäß Anspruch 12 verwendet.

14. Verfahren gemäß Anspruch 13, dadurch gekennzeichnet, dass die chlorierten Alkane unter den Chlorethanen und den Chlorpropanen ausgewählt sind.

## Claims

1. Process for the preparation of a catalyst including a metal of group VIII and a metal of group Ib on a support including an inorganic oxide, characterized in that:
- in a first stage a mixture is dissolved in an organic solvent, including an alkoxide precursor of the inorganic oxide, a complex of a compound of the metal of group VIII and of a difunctional complexing compound corresponding to the general formula A-(CHR²)ₐ-Si(OR¹)₃ in which
a is an integer from 0 to 8,
R¹ is an alkyl chain containing from 1 to 8 carbon atoms,
R² is a hydrogen atom or an alkyl chain containing from 1 to 4 carbon atoms,
A denotes an electron-donor group capable of complexing the metal of group VIII,
and a complex of a compound of the metal of group Ib and of a difunctional complexing compound corresponding to the general formula B-(CHR^{2'})_{a'}-Si(OR^{1'})₃ in which
a' is an integer from 0 to 8,
R^{1'} is an alkyl chain containing from 1 to 8 carbon atoms,
R^{2'} is a hydrogen atom or an alkyl chain containing from 1 to 4 carbon atoms,
B denotes an electron-donor group capable of complexing the metal of group Ib;
- in a second stage the mixture is hydrolysed in order to form a gel; and
- in a third stage the gel is dried.

2. Process according to Claim 1, characterized in that the metal of group VIII is palladium and the metal of group Ib is silver.

3. Process according to Claim 1 or 2, characterized in that the electron-donor group A is a group selected from those of general formula R⁶R⁵N-(CHR⁴)_{b}-N(R⁷)- in which R⁴, R⁵, R⁶ and R⁷ are, independently, selected from a hydrogen atom or an alkyl chain containing from 1 to 4 carbon atoms and b is an integer from 1 to 4, and the electron-donor group B is a group selected from those of general formula R^{6'}R^{5'}N-(CHR^{4'})_{b'}-N(R^{7'})- in which R^{4'}, R^{5'}, R^{6'} and R^{7'} are, independently, selected from a hydrogen atom or an alkyl chain containing from 1 to 4 carbon atoms, and b' is an integer from 1 to 4, and those of general formula N(R^{3'})₂- in which R^{3'} is a hydrogen atom or an alkyl chain containing from 1 to 4 carbon atoms.

4. Process according to Claim 3, characterized in that the metal of group VIII is palladium and the compound complexing this metal corresponds to the general formula R⁶R⁵N-(CHR⁴)_{b}-NH-(CHR²)ₐ-Si(OR¹)₃ in which
a is an integer from 1 to 4,
b is an integer from 1 to 3,
R¹ is an alkyl chain containing from 1 to 4 carbon atoms,
R², R⁴, R⁵ and R⁶ are, independently, chosen from a hydrogen atom and a methyl or ethyl chain.

5. Process according to Claim 4, characterized in that the compound of the metal of group VIII is palladium acetylacetonate and the compound complexing this metal corresponds to the general formula NH₂-(CH₂)₂-NH-(CH₂)₃-Si(OR¹)₃.

6. Process according to any one of Claims 3 to 5, characterized in that the metal of group Ib is silver and the compound complexing this metal corresponds to the general formula R^{6'}R^{5'}N-(CHR^{4'})_{b'}-NH-(CHR^{2'})_{a'}-Si(OR^{1'})₃ or to the general formula N(R^{3'})₂-(CHR^{2'})_{a'}-Si(OR^{1'})₃ in which
a' is an integer from 1 to 4,
b' is an integer from 1 to 3,
R^{1'} is an alkyl chain containing from 1 to 4 carbon atoms,
R^{2'}, R^{3'}, R^{4'}, R^{5'} and R^{6'} are, independently, selected from a hydrogen atom and a methyl or ethyl chain.

7. Process according to Claim 6, characterized in that the compound of the metal of group Ib is silver acetate and the compound complexing this metal corresponds to the general formula NH₂-(CH₂)₂-NH-(CH₂)₃-Si(OR^{1'})₃ or to the general formula NH₂-(CH₂)₃-Si(OR^{1'})₃.

8. Process according to any one of Claims 1 to 7, characterized in that the alkoxide precursor of the inorganic oxide is selected from silicon, aluminium, titanium and zirconium alkoxides.

9. Process according to any one of Claims 1 to 8, characterized in that the respective quantities of the complexes and of the alkoxide precursor are adjusted so as to obtain a catalyst containing from 0.05 % to 15 % by weight of the metal of group VIII and from 0.05 % to 15 % by weight of the metal of group Ib.

10. Process according to any one of Claims 1 to 9, characterized in that the respective quantities of the complexes are adjusted so as to obtain a catalyst in which the weight ratio of the metal of group VIII to the metal of group Ib is from 0.1 to 10.

11. Process according to any one of Claims 1 to 10, characterized in that in the third stage the gel is dried at a pressure which is lower than atmospheric pressure in order to obtain a xerogel.

12. Catalyst including a metal of group VIII and a metal of group Ib on a support including an inorganic oxide, capable of being obtained by means of the process according to any one of Claims 1 to 11.

13. Process for conversion of chloroalkanes into alkenes containing less chlorine, by means of hydrogen, characterized in that a catalyst according to Claim 12 is used.

14. Process according to Claim 13, characterized in that the chloroalkanes are chosen from chloroethanes and chloropropanes.
